Europäisches Patentamt

(19) European Patent Office

Office européen des brevets

(11) Numéro de publication : **0 030 902**
**B1**

(12) **FASCICULE DE BREVET EUROPÉEN**

(45) Date de publication du fascicule du brevet :
21.03.84

(51) Int. Cl.³ : **D 04 B 21/20**

(21) Numéro de dépôt : 80420134.1

(22) Date de dépôt : 09.12.80

(54) **Bande maillée extensible en tout sens.**

(30) Priorité : 12.12.79 FR 7930900

(43) Date de publication de la demande :
24.06.81 Bulletin 81/25

(45) Mention de la délivrance du brevet :
21.03.84 Bulletin 84/12

(84) Etats contractants désignés :
AT BE CH DE GB IT LI LU NL SE

(56) Documents cités :
FR-A- 818 470
FR-A- 2 171 104
FR-A- 2 241 645
FR-A- 2 451 412
US-A- 3 570 482
US-A- 3 747 374

(73) Titulaire : **MOLINIER S.A. société anonyme
chemin du Siccard
F-42340 Veauche (FR)**

(72) Inventeur : **L'inventeur a renoncé à sa désignation**

(74) Mandataire : **Buttet, Roger et al
"Cabinet Charras" 3, Place de l'Hôtel-de-Ville
F-42000 Saint-Etienne (FR)**

Jouve, 18, rue St-Denis, 75001 Paris, France

Bande maillée extensible en tous sens

L'invention concerne une bande maillée extensible en tous sens.

On connaît un tricot-chaîne (FR-A-818 470 MICHEL), composé de fils de chaîne non extensibles associés à des fils de chaîne élastiques et à deux fils de trame différents. Le tricot obtenu est constitué de bandes juxtaposées comportant alternativement une trame extensible et une trame élastique, ceci sans aucun maillage.

On connaît également un bandage chirurgical (Brevet US-A-3 570 482 EMOTO), composé d'un fil de chaîne en matière non élastique, d'un fil de trame en matière non élastique, et un fil élastique polyuréthanne, tricoté le long de la côte dans tout ou partie des points de chaîne, de manière à diviser le bandage dans la longueur, par arrachage des fils de trame.

De tels tricot-chaîne ou bandage ne permettent pas de réaliser des bandes maillées extensibles en tous sens, s'attachant à des aspects particuliers de leurs utilisations, en améliorant l'effet de contention, et d'élasticité dans tous les sens, pour obtenir, après chaque allongement, un effet de rétraction constant et une permanence de sa valeur et de son action dans le temps. On a aussi voulu améliorer l'aspect ou présentation de la bande.

L'invention telle que caractérisée dans les revendications résout le problème en exécutant la bande maillée extensible en tous sens, sur des métiers à tricoter du type Rachel ou chaîne, avec une combinaison des fils qui la composent et qui sont, dans le sens longitudinal, des chaînettes formées exclusivement de fils extensibles, et dans le sens transversal, en trame, des fils extensibles et des fils inextensibles alternés qui relient les chaînettes selon une armure ou structure tissée, de façon telle que chaque chaînette soit liée aux deux sortes de fils de trame, dans les mêmes conditions que les autres chaînettes, avec interpénétration réciproque de chaque fil de trame reliant plusieurs chaînettes, par rapport aux fils de trame voisins.

Ces caractéristiques et d'autres encore ressortiront bien de la suite de la description.

Pour fixer l'objet de l'invention, sans toutefois le limiter, aux figures des dessins annexés :

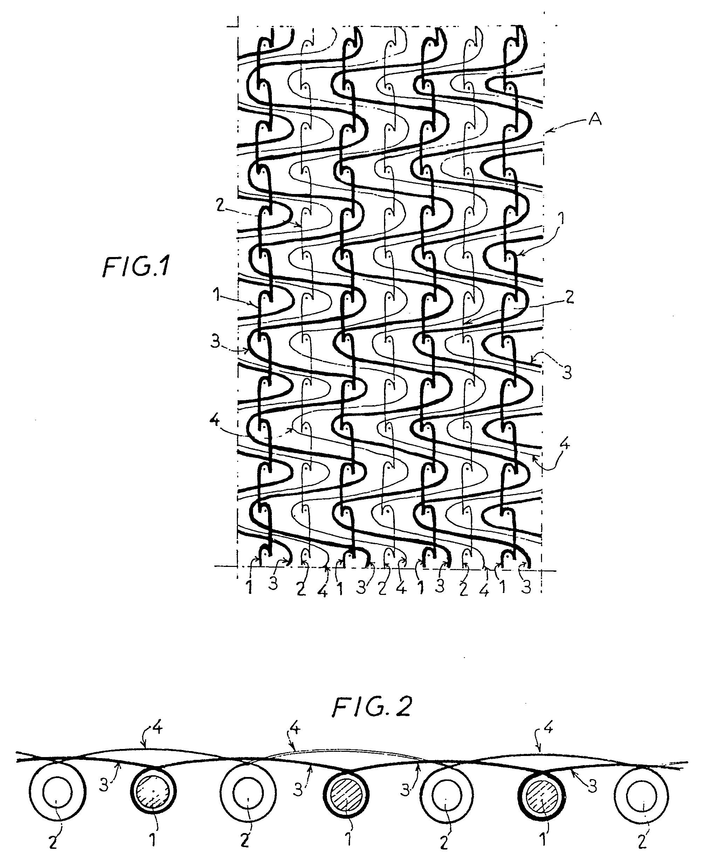

La figure 1 est à une échelle largement agrandie par rapport à la réalité, une première forme de réalisation d'une bande maillée.

La figure 2 est à plus grande échelle, une vue en plan de la bande illustrée à la figure 1.

Selon la présente invention et dans l'exemple nullement limitatif illustré aux dessins, cette bande A comprend essentiellement une combinaison de fils qui sont, dans le sens longitudinal, des fils de chaînettes élastiques 1-2, alternés, de natures différentes. Les fils identiques de chaque chaînette peuvent être des fils simples ou composés de plusieurs fils élastiques pour chaque chaînette. Les fils élastiques des chaînettes peuvent être : des fils texturés présentant une élasticité artificielle de formes (fils frisés ou fils mousse), des fils en élastomère, des fils de gomme nue ou guipée, des fils crêpés, coton ou laine.

Dans ce cas, les fils 1 sont par exemple, des fils en élastomère guipé ou non, et les fils 2 sont des fils « mousse ».

Toutes les aiguilles du métier Rachel ou chaîne, sont en travail. Les barres à passettes sont enfilées dans l'exemple illustré, en mélangeant 1/1, c'est-à-dire que l'on enfile alternativement un fil 1 et un fil 2 sur les barres à passettes du métier.

Il est précisé que, selon l'invention, l'enfilage des fils de chaîne sur les barres à passettes, peut aussi s'effectuer en mélangeant toujours alternativement mais dans d'autres rapports, par exemple dans le rapport 2/2, c'est-à-dire deux fils élastiques de même nature (fils en élastomère, guipés par exemple) et deux fils élastiques d'une autre nature (fils mousse par exemple). Ou bien l'enfilage pourrait s'effectuer dans le rapport 3/3, ou encore dans le rapport 2/1 (c'est-à-dire deux fils élastiques suivis d'un fil élastique de nature différente, puis de deux fils élastiques, etc...

Il pourrait aussi y avoir plus de deux sortes de fils élastiques alternés.

D'autre part, dans le sens transversal, la bande maillée A est constituée par une combinaison de trames de fils extensibles 3 et de fils inextensibles 4, alternés.

Les fils élastiques 3 peuvent être simples ou composés de plusieurs fils élastiques pour chaque trame : les fils élastiques 3 peuvent être : des fils texturés présentant une élasticité artificielle de forme (fils frisés ou fils mousse), des fils en élastomère, des fils de gomme nue ou guipée...

Dans l'exemple illustré, les fils 3 sont par exemple, des fils en gomme ou en élastomère guipé.

Les fils 4 qui peuvent être simples ou composés, sont des fils en fibranne, en coton ou autres fils inextensibles.

Les barres à passettes sont enfilées, pour les trames, en mélangeant, selon l'exemple illustré, dans le rapport 1/1, comme pour les chaînes, c'est-à-dire en enfilant alternativement un fil 3 et un fil 4 sur les barres à passettes.

De la même façon que pour les chaînes, on peut alterner les fils 3 et 4 dans d'autres rapports : 2/2, 2/1, 1/2, 3/3...

Comme illustré, les fils de trame inextensibles, doublent constamment les fils extensibles. Chaque chaînette est liée aux deux sortes de fils de trame (extensible et inextensible) dans les mêmes conditions que les autres chaînettes, et il y a interpénétration réciproque de chaque fil de trame extensible ou inextensible reliant plusieurs chaînettes (trois chaînettes, à titre d'exemple non limitatif, aux figures des dessins), par rapport aux fils de trame voisins.

La bande maillée extensible ainsi réalisée, améliore les effets élastiques, l'effet de contention contrôlé, en particulier par l'alternance des fils de trame, les fils de trame élastiques assurant l'élasticité, notamment dans la largeur, tandis que les fils de trame inextensible participent au confort et à la douceur de la bande. En chaîne, l'alternance des fils élastiques de natures diverses, contribue à l'équilibrage et à la tenue de la bande. Plus particulièrement, les trames et chaînettes alternées et de natures diverses, donnent un équilibre à la bande avec une bonne extensibilité dans tous les sens, et aussi une contention suffisante et contrôlée, avec, après les allongements élastiques, un effet de rétraction constant et permanent dans le temps. L'aspect de la bande est aussi amélioré.

## Revendications

1. Bande maillée extensible en tout sens (A), exécutée sur des métiers à tricoter du type Rachel ou chaîne, comprenant une combinaison de fils qui sont, dans le sens longitudinal, des chaînettes (1, 2), et dans le sens transversal, en trame, des fils extensibles (3) et des fils inextensibles (4) alternés, qui relient les chaînettes selon une armure ou structure tissée, caractérisée en ce que les dites chaînettes sont composées exclusivement de fils extensibles (1, 2) et en ce que les fils de trame sont passés de telle sorte que chaque chaînette soit liée aux deux sortes de fils de trame (3, 4) dans les mêmes conditions que les autres chaînettes, avec interpénétration réciproque de chaque fil de trame reliant plusieurs chaînettes, par rapport aux fils de trame voisins.

2. Bande maillée selon 1, caractérisée en ce que dans le sens longitudinal, les fils de chaînettes élastiques (1) (2) sont en alternance, des fils élastiques, de natures différentes : fils texturés, fils en élastomère guipés ou non, fils de gomme nue ou guipée, fils crêpés, en coton ou en laine.

3. Bande maillée selon 1 et 2, caractérisée en ce que les fils élastiques des chaînettes sont des fils simples guipés ou non, ou composés de plusieurs fils élastiques pour chaque chaînette.

4. Bande maillée selon 1, 2 ou 3, caractérisée en ce que pour l'exécuter, toutes les aiguilles du métier Rachel ou chaîne, sont en travail, les barres à passettes étant enfilées en mélangeant 1/1, ou bien dans d'autres rapports, mais toujours en alternance.

5. Bande maillée selon 1, caractérisée en ce que pour l'exécuter, toutes les aiguilles du métier Rachel ou chaîne, sont en travail, les barres à passettes étant enfilées en mélangeant 1/1, ou bien dans d'autres rapports, mais toujours en alternance des trames extensibles (3) et inextensibles (4).

## Claims

1. Stitch bandage (A) which is extensible in all directions and manufactured on knitting machines of the warp or Raschel type, including a combination of yarns which in the lengthwise direction are the chains (1, 2) and which are, in the transverse direction, weftwise, alternate extensible yarns (3) and not extensible yarns (4), connecting the chains in accordance with a weave or a woven structure, characterized in that said chains are comprised exclusively of extensible yarns (1, 2), and also in that the weft yarns are passed so that each chain will be tied with two kinds of weft yarns (3, 4) under the same conditions as the other chains, with mutual interpenetration of each weft yarn connecting a plurality of chains, relative to the adjacent weft yarns.

2. Stitch bandage as claimed in Claim 1, characterized in that the elastic chain yarns (1) (2), in the longitudinal direction, are alternately elastic yarns of different nature : textured yarns, covered or not covered elastomeric yarns, exposed or covered rubber yarns, crimped yarns, of cotton or wool.

3. Stitch bandage as claimed in Claims 1 and 2, characterized in that the elastic yarns of the chains are covered or not covered simple yarns, or are comprised of several elastic yarns for each chain.

4. Stitch bandage as claimed in Claims 1, 2 or 3, characterized in that, for manufacturing this bandage, all the needles of the warp or Raschel machine are at work, the heddle hook bars being threaded by mixing in the 1/1 or other ratio, but always alternately.

5. Stitch bandage as claimed in Claim 1, characterized in that, for manufacturing this bandage, all the needles of the warp or Raschel machine are at work, the heddle hook bars being threaded by mixing in the 1/1 or other ratio, but always by alternating the extensible wefts (3) and the non-extensible wefts (4).

## Ansprüche

1. Nach jeder Richtung ausdehnbare Maschenbinde (A), die auf Wirkmaschinen vom Typ der Ketten- oder Raschelmaschinen gefertigt wird und eine Kombination von Garnen aufweist, die in der Längsrichtung die Ketteln (1, 2) und in der Querrichtung, einschlagsweise, die abwechselnden ausdehnbare Garne (3) und nichtausdehnbare Garne (4) sind, die die Ketteln entsprechend einer Bindung oder einer gewebten Gliederung verbinden, dadurch gekennzeichnet, dass die genannten Ketteln ausschliesslich aus den ausdehnbaren Garnen (1, 2) bestehen, und dass die Einschlagsgarne in der Weise eingefädelt sind, dass jede Kettel mit den zweien Arten von Einschlagsgarnen (3, 4) in denselben Bedingungen wie bei den anderen Ketteln, mit beiderseitiger Zwischeneindringung jedes hinsichtlich der angrenzenden Einschlagsgarne mehrere Ketteln verbindenden Einschlagsgarns verbunden wird.

2. Maschenbinde nach Anspruch 1, dadurch gekennzeichnet, dass in der Längsrichtung, die elastischen Kettelgarne abwechselnd elastische Garne unterschiedlicher Art : Texturgarne, Elastomergarne mit oder ohne Umspinnung, Gummigarne mit oder ohne Umspinnung, Kreppgarne aus Baumwolle oder Wolle sind.

3. Maschenbinde nach Anspruch 1 und 2, dadurch gekennzeichnet, dass die elastischen Garne der Ketteln einfache Garne mit oder ohne Umspinnung oder aus für jede Kettel mehreren elastischen Fäden bestehende Garne sind.

4. Maschenbinde nach Anspruch 1, 2 oder 3, dadurch gekennzeichnet, dass zur Fertigung dieser Binde alle die Nadeln der Ketten- oder Raschelwirkmaschine eingesetzt sind, wobei die Einziehhakenstangen mit Mischung 1/1 oder in anderen Verhältnissen, jedoch stets abwechselnd, eingefädelt werden.

5. Maschenbinde nach Anspruch 1, dadurch gekennzeichnet, dass zur Fertigung dieser Binde alle die Nadeln der Ketten- oder Raschelwirkmaschine eingesetzt sind, wobei die Einziehhakenstangen mit Mischung 1/1 oder in anderen Verhältnissen, jedoch stets abwechselnd hinsichtlich der ausdehnbaren (3) und nicht ausdehnbaren (4) Einschläge eingefädelt werden.

FIG.1

FIG.2